(19) Europäisches Patentamt / European Patent Office / Office européen des brevets

(11) **EP 4 005 998 A1**

(12) **EUROPEAN PATENT APPLICATION**

(43) Date of publication:
01.06.2022 Bulletin 2022/22

(21) Application number: 21202787.4

(22) Date of filing: 15.10.2021

(51) International Patent Classification (IPC):
C04B 35/486 (2006.01)     C04B 35/488 (2006.01)
C04B 35/64 (2006.01)      A61C 13/00 (2006.01)
C04B 41/00 (2006.01)      C04B 41/50 (2006.01)
C04B 41/85 (2006.01)

(52) Cooperative Patent Classification (CPC):
(C-Sets available)
A61K 6/16; A61C 13/0022; A61C 13/083;
A61K 6/802; A61K 6/818; A61K 6/822;
C04B 35/486; C04B 35/488; C04B 35/64;
C04B 41/009; C04B 41/5009; C04B 41/85;
C04B 2111/00836; C04B 2235/3217;
C04B 2235/3225;                      (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 16.10.2020   JP 2020174307

(71) Applicant: Shofu Inc.
Kyoto 605-0983 (JP)

(72) Inventors:
• NONAKA, Kazumichi
Kyoto, 605-0983 (JP)
• TAKAHASHI, Shuhei
Kyoto, 605-0983 (JP)

(74) Representative: Eisenführ Speiser
Patentanwälte Rechtsanwälte PartGmbB
Postfach 31 02 60
80102 München (DE)

(54) **DENTAL ZIRCONIA MILL BLANK FOR CUTTING AND MACHINING INCLUDING INDIUM AND YTTRIUM**

(57)   [Problem]
A technique for imparting high translucency which is similar to an enamel of a natural tooth to a zirconia sintered body, has been required.
[Solution]
To provide a zirconia mill blank for dental cutting and machining, containing, an yttrium compound and an indium compound as stabilizers, wherein, an amount of the yttrium compound is within a range of 3.0 mol% to 6.0 mol% in terms of oxide, an amount of the indium compound is within a range of 0.2 mol% to 3.0 mol% in terms of oxide, and a total amount of the yttrium compound and the indium compound is within a range of 5.5 mol% to 7.0 mol% in terms of oxide.

EP 4 005 998 A1

(52) Cooperative Patent Classification (CPC): (Cont.)
C04B 2235/3246; C04B 2235/3272;
C04B 2235/3286; C04B 2235/443;
C04B 2235/604; C04B 2235/612;
C04B 2235/616; C04B 2235/6562;
C04B 2235/6567; C04B 2235/661;
C04B 2235/9653; C04B 2235/9661

C-Sets
**C04B 41/009, C04B 35/48;**
**C04B 41/5009, C04B 41/4535, C04B 2103/0021**

**Description**

BACKGROUND OF THE INVENTION

Field of the Invention

The present invention relates to a zirconia mill blank for dental cutting and machining and a preparing method thereof.

Description of the Related Art

In recent years, techniques to prepare a prosthesis device by the cutting and machining which uses the dental CAD/CAM system has been spread rapidly and therefore it has been becoming possible to easily prepare prosthetic devices by cutting and machining the mill blanks which are made of ceramic materials such as a zirconia, an alumina and a lithium disilicate glass, or resin materials such as an acrylic resin and a hybrid resin.

In particular, the zirconia has been clinically applied in various cases because of its high strength. On the other hand, a zirconia final fired body which is final fired and can be used in the oral cavity (hereinafter, referred to as "zirconia sintered body") has a very high hardness, and therefore cannot be cut and machined by using a dental CAD/CAM system. Thus, a zirconia which is not finally fired but is calcined at a low firing temperature to adjust to a hardness that enables to cut and machine has been used as a zirconia mill blank for dental cutting and machining.

When a zirconia was initially applied as a dental material, the zirconia had high strength but low translucency, and therefore it was mainly used as a coping or a frame.

In recent years, because of the development of zirconia with improved translucency (high translucency zirconia), its usages has been expanding from a molar tooth to a full crown of a front tooth.

However, even if using high translucency zirconia, the translucency is insufficient to reproduce an enamel of a natural tooth. Therefore, especially in cases where aesthetic property is required, a natural restoration has been prepared by building porcelain on a zirconia.

In this situation, it has been desired to prepare a more natural restoration with a full contour zirconia, and therefore, it has been necessary to develop a zirconia having excellent translucency.

Patent Document 1 discloses a zirconia mill blank for dental cutting and machining prepared by using a zirconia powder containing 3 mol% of yttrium with a reduced alumina content and a zirconia sintered body prepared from the zirconia mill blank. Since the translucency is improved in the sintered body while maintaining high strength, the sintered body is clinically applied in a long span bridge of 4 or more units, a molar part full crown and the like. However, since translucency is insufficient in the sintered body, it has been difficult to apply to the case where high aesthetic property is required such as a front tooth portion.

Relevant References

Patent Literature

[Patent document 1] Japanese Unexamined Patent Application Publication No. 2010-150063

SUMMARY OF THE INVENTION

Technical Problem

A technique for imparting high translucency which is similar to an enamel of a natural tooth to a zirconia sintered body, has been required.

Solution to Problem

The present inventors made a study on a zirconia mill blank for dental cutting and machining which may impart high translucency which is similar to an enamel of a natural tooth to a zirconia sintered body.

The zirconia mill blank for dental cutting and machining of the present invention is a zirconia mill blank for dental cutting and machining, containing, an yttrium compound and an indium compound as a stabilizer, wherein, an amount of the yttrium compound is within a range of 3.0 mol% to 6.0 mol% in terms of oxide, an amount of the indium compound is within a range of 0.2 mol% to 3.0 mol% in terms of oxide, and a total amount of the yttrium compound and the indium compound is within a range of 5.5 mol% to 7.0 mol% in terms of oxide. In the present invention, the remainder other than specified component as the components constituting of the zirconia mill blank for dental cutting and machining of the present invention can be composed of zirconia ($ZrO_2$). Further, an amount of zirconia ($ZrO_2$) can be within a range of 90 mol% to 94.5 mol% in terms of oxide. Furthermore, a total of the yttrium concentration and the indium concentration may be within a range of 6.0 mol% to 6.7 mol%.

In the zirconia mill blank for dental cutting and machining of the present invention, a sintered body of the zirconia mill blank for dental cutting and machining may have a contrast ratio of 0.68 or less for a sample having a thickness of 1 mm.

In the zirconia mill blank for dental cutting and machining of the present invention, the zirconia mill blank for dental cutting and machining may further contain a colorant, and a sintered body of the zirconia mill blank for dental cutting and machining may have a contrast ratio of 0.68 or less for a sample having a thickness of 1 mm.

In the zirconia mill blank for dental cutting and machining of the present invention, the zirconia mill blank for dental cutting and machining may contain no colorant, and a sintered body of the zirconia mill blank for dental cutting and machining may have a contrast ratio of 0.65 or less for a sample having a thickness of 1 mm.

The present invention also provides a dental prosthetic device prepared by sintering the zirconia mill blank for dental cutting and machining of the present invention.

Advantageous Effects of Invention

The zirconia mill blank for dental cutting and machining of the present invention may impart high translucency which is similar to an enamel of a natural tooth to a zirconia sintered body.

DETAILED DESCRIPTION OF THE PREFERRED EMBODIMENTS

The zirconia mill blank for dental cutting and machining of the present invention is a zirconia mill blank for dental cutting and machining, containing, an yttrium compound and an indium compound as a stabilizer, wherein, an amount of the yttrium compound is within a range of 3.0 mol% to 6.0 mol% in terms of oxide, an amount of the indium compound is within a range of 0.2 mol% to 3.0 mol% in terms of oxide, and a total amount of the yttrium compound and the indium compound is within a range of 5.5 mol% to 7.0 mol% in terms of oxide. In the present invention, the remainder other than specified component as the components constituting of the zirconia mill blank for dental cutting and machining of the present invention can be composed of zirconia ($ZrO_2$). Further, an amount of zirconia ($ZrO_2$) can be within a range of 90 mol% to 94.5 mol% in terms of oxide.

A content of the indium compound is more preferably within a range of 0.4 mol% to 1.3 mol% in terms of indium oxide ($In_2O_3$). When the amount of indium oxide is less than 0.2 mol%, sufficient translucency may not be imparted to a zirconia sintered body. On the other hand, when the amount of indium oxide exceeds 3.0 mol%, sufficient translucency may not be imparted to a zirconia sintered body.

There is no limitation on the state of the indium compound in the zirconia mill blank for dental cutting and machining of the present invention before becoming a zirconia sintered body. Specifically, it may be solid solved in the zirconia, or may exist as a crystal or an amorphous as an indium compound, which are different from the zirconia.

Any known indium compounds can be used as the indium compound without any limitation. Specific examples of the indium compound used in the present invention include oxides, halides, nitrates, sulfates, organic acid salts of indium, and the like. **More specific examples include indium oxide, indium nitrate, indium chloride,**

**indium sulfate, and the like.**

As a method of adding an indium compound to the zirconia mill blank for dental cutting and machining in the present invention, a method which can add uniformly a specific amount of the indium compound to the zirconia mill blank for dental cutting and machining is preferable. For example, a method which comprises adding an indium compound in preparing a zirconia particle may be used, or a method which comprises immersing a zirconia mill blank for cutting and machining in a solution containing an indium compound may be used.

When the method which comprises immersing a zirconia mill blank for cutting and machining in a solution containing an indium compound is used, any solvents can be used for the indium solution, such as water, alcohol, an organic solvent and the like. Water, ethanol and a mixture thereof are particularly preferable since it is easily available and is easy to handle.

A method for preparing the solution containing an indium compound is not particularly limited, and there is no problem at all with any preparation method as long as the indium compound is dissolved in the solvent.

There is no limitation in a specific atmosphere in which the zirconia mill blank for dental cutting and machining is permeated with a solution containing an indium compound, and there is no problem in a normal pressure atmosphere, a reduced pressure atmosphere and a pressurized atmosphere. From the viewpoint of shortening the preparation time, a reduced pressure atmosphere or a pressurized atmosphere is preferable as the surrounding environment because of promoting the permeation of the solution containing an indium compound. In addition, it is effective for shortening the time of the step in which the solution containing an indium compound infiltrates into a space which is in inside of the zirconia mill blank for dental cutting and machining and communicates with the outside of the zirconia mill blank for dental cutting and machining, that the operation of returning to normal pressure after the pressure reduction operation (pressure reduction/normal pressure operation) or the operation of returning to normal pressure after the pressuring operation (pressuring/normal pressure operation) is repeated multiple times.

The time for immersing the zirconia mill blank for cutting and machining in the solution containing the indium compound is not determined unconditionally and can be adjusted appropriately based on the relative density and the molded body size of the zirconia mill blank for cutting and machining and the degree of infiltration of and the method for immersing of the solution containing the indium compound and the like. For example, the time for immersing is usually 1 to 120 hours in the case of immersing, the time for immersing is usually 0.5 to 12 hours in the case of immersing under reduced pressure, and the time for immersing is usually 0.2 to 6 hours in the case of contacting under pressurization.

It is more preferable that an amount of yttrium compound contained in the zirconia mill blank for dental cutting and machining in the present invention is within a range of 4.0 mol% to 5.5 mol% in terms of yttrium oxide ($Y_2O_3$). When the amount of yttrium oxide is less than 3.0 mol%, it is not preferable because sufficient translucency may not be imparted after final firing of the zirconia. When the amount of yttrium oxide exceeds 6.0 mol%, it is not preferable because the translucency of the zirconia final fired body tends to be lowered. The final firing is a firing step for preparing a prosthetic device which can be used in the oral cavity, and the final fired body is the one which is prepared by similar firing step to the prosthetic device which can be used in the oral cavity.

It is more preferable that the total amount of the yttrium compound and the indium compound contained in the zirconia mill blank for dental cutting and machining in the present invention is within a range of 6.0 mol% to 6.7 mol% in terms of oxide. When the total amount of the yttrium compound and the indium compound is less than 5.5 mol%, it is not preferable because sufficient translucency may not be imparted after final firing of the zirconia. When the total amount of the yttrium compound and the indium compound exceeds 7.0 mol%, it is not preferable because sufficient translucency may not be imparted after final firing of the zirconia.

In the present invention, it is preferable to satisfy any one of the three requirements that the yttrium compound is within a range of 4.0 mol% to 5.5 mol% in terms of yttrium oxide, the indium compound is within a range of 0.4 mol% to 1.3 mol% in terms of indium oxide and the total amount of the yttrium compound and the indium compound is within a range of 6.0 mol% to 6.7 mol% in terms of oxide, more preferable to satisfy the two requirements, and most preferable to satisfy the three requirements. As more requirements are satisfied, the contrast ratio becomes lower and the translucency is improved.

It is preferable that the primary particle diameter of a zirconia powder used for preparing the zirconia mill blank for dental cutting and machining in the present invention is within a range of 1 to 500 nm. When the primary particle diameter is less than 1 nm, there is a tendency that it is difficult to impart sufficient strength, although the translucency of the zirconia sintered body is improved. On the other hand, when the primary particle diameter is more than 500 nm, there is a tendency that it is difficult to impart sufficient strength to the zirconia sintered body.

It is preferable that the zirconia mill blank for dental cutting and machining of the present invention contains a colorant. Specific examples thereof include an inorganic colorant. More specific examples thereof include iron oxide, erbium, cobalt, manganese, chromium, and rare earth elements. Iron oxide may be added for imparting a yellow color and erbium may be added for imparting a red color. In addition to these colorants, it is preferable that the element such as cobalt, manganese and chromium is used in combination for a color tone adjustment. In the present invention, it is preferable to color the tooth color by including the colorant.

The zirconia mill blank for dental cutting and machining of the present invention may contain a sintering aid. Specifically, for the purpose of improving the sinterability and suppressing low temperature deterioration, it is preferable to contain 0.01 to 0.3 mass% of alumina. When the amount of alumina is less than 0.01 mass%, there is a tendency that it may be difficult to obtain sufficient characteristics even after final firing and therefore sufficient strength and translucency may be not imparted. On the other hand, when the amount of alumina exceeds 0.3 mass%, there is a tendency that it may be difficult to impart sufficient translucency although strength of the zirconia sintered body is improved.

It is preferable that the relative density of the zirconia sintered body prepared by firing the zirconia mill blank for dental cutting and machining of the present invention at within a range of 1450 °C to 1600 °C is 98% or more of the theoretical density. The relative density is determined by the measured density/the theoretical density. When the relative density is less than 98%, the strength and translucency tend to be lowered.

It is preferable that a crystal phase of the zirconia mill blank for dental cutting and machining of the present invention is tetragonal and/or cubic. When the crystal phase is monoclinic phase, it is not preferable because sufficient translucency may be not imparted even after final firing.

A preparing method of the zirconia mill blank for dental cutting and machining of the present invention is not particularly limited, and any known preparing methods can be used without any problem. Specifically, it is preferable to be prepared by molding a zirconia powder by press molding. Furthermore, it is more preferable to be prepared by a multilayer molding in which zirconia powders having different color tones or compositions are press-molded in multiple stages.

The zirconia mill blank for dental cutting and machining of the present invention is preferably subjected to isostatic pressing by cold isostatic pressing (CIP treatment) after the press molding.

The maximum load pressure of CIP treatment in the present invention is preferably 50 Mpa or more. When the maximum load pressure is less than 50 MPa, there is a case where sufficient translucency and strength may not bet imparted to the zirconia sintered body.

A calcination temperature of the zirconia mill blank for dental cutting and machining of the present invention is preferably within a range of 800 to 1200 °C. When the calcination temperature is less than 800 °C, because Vickers hardness and/or bending strength become too low and therefore there is a tendency that chipping and breakage easily occur in the cutting and machining. On the other hand, when the calcination temperature is more than 1200 °C, because Vickers hardness and/or bending strength become too high and therefore there is a tendency that a milling bar of a milling machine is heavily consumed to raise a running cost.

In this way, a zirconia mill blank for dental cutting and machining can be prepared by the preparing method of the present invention. The prepared zirconia mill blank for dental cutting and machining is severed, cut, and polished so as to have a desired size as necessary.

A method for final firing the zirconia mill blank for dental cutting and machining of the present invention is not particularly limited, but a simple and preferred method is to firing at normal pressure. The firing temperature is not particularly limited, but is preferably within a range of 1450 to 1600 °C, particularly preferably within a range of 1500 to 1600 °C. The holding time at the firing temperature is not particularly limited, but is preferably within a range of 1 minute to 12 hours, and particularly preferably within a range of 2 to 4 hours. The temperature increase rate is not particularly limited, but is preferably within a range of 1 to 400 °C/min, and more preferably within a range of 3 to 100 °C/h.

In the present invention, it is preferable that a sintered body which is prepared by final firing the zirconia mill blank for dental cutting and machining has a contrast ratio of 0.68 or less for a sample having a thickness of 1 mm.

In the present invention, it is preferable that a sintered body which is prepared by final firing the zirconia mill blank for dental cutting and machining which contains a colorant has a contrast ratio of 0.68 or less for a sample having a thickness of 1 mm.

In the present invention, it is preferable that a sintered body which is prepared by final firing the zirconia mill blank for dental cutting and machining which contains no colorant has a contrast ratio of 0.65 or less for a sample having a thickness of 1 mm.

The kind of a prosthesis device prepared by cutting and machining the zirconia mill blank for dental cutting and machining according to the present invention is not limited particularly, and there is no problem at all even if the prosthesis device is any of an inlay, an onlay, a veneer, a crown, a bridge and the like. Therefore, a shape of a zirconia mill blank for dental cutting and machining which is cut and machined for preparing a prosthesis device is not limited particularly, and any zirconia mill blank for dental cutting and machining can be used even if the zirconia mill blank for dental cutting and machining has any shape such as a block shape corresponding to an inlay, an onlay, a veneer, a crown and the like and a disk shape corresponding to a bridge.

[Examples]

Hereinafter, the present invention is described by way of Examples in more detail, and specifically, but the present invention is not limited to these Examples.

[Measurement of indium oxide and yttrium oxide content (mol%)]

The test specimen for evaluating the indium and yttrium contents was prepared by cutting and machining each zirconia mill blank into a round plate shape ($\varphi$14 mm × 1.6 mm). The amount of indium and yttrium on each of the upper surface and the lower surface of each test specimen was measured by using a fluorescent X-ray analysis device (manufactured by Rigaku Corporation), and the average value of each of the upper surface and the lower surface was defined as the indium and yttrium contents. Further, the indium and yttrium contents (mol%) are shown in terms of oxide.

[Sintering conditions]

The zirconia mill blank for dental cutting and machining was cut and machined into a predetermined shape, and fired in a firing furnace (firing temperature: 1550 °C, temperature increase rate: 5 °C/min, holding time: 120 minutes) to prepare a zirconia sintered body.

[Evaluation of Translucency]

The test specimen for evaluating the translucency was prepared by cutting and machining the zirconia mill blank for dental cutting and machining into a round plate shape ($\varphi$14 mm $\times$ 1.6 mm). Each test specimen was sintered in a firing furnace. Then, each test specimen was adjusted to have the thickness (1.0 mm) with a surface grinder. The translucency was evaluated by measuring the contrast ratio. The contrast ratio was measured by using a spectrocolorimeter (manufactured by Konica Minolta). In the following formula, Yw is the value Y measured by placing the white plate behind the test specimen, and Yb is the value Y measured by placing the black plate behind the test specimen. The contrast ratio was calculated from the following formula.

When the contrast ratio value is close to zero, the materials are seen as transparency. When the contrast ratio value is close to 1, the materials are seen as opaqueness.

$$\text{Formula: The contrast ratio} = \text{Yb} / \text{Yw}$$

Further, the following ABC score was used for the evaluation using the contrast ratio.

A: The contrast ratio of sintered body containing no colorant $\leq$ 0.65
B: 0.65 < The contrast ratio of sintered body containing no colorant $\leq$ 0.68
C: The contrast ratio of sintered body containing no colorant > 0.68:

A: The contrast ratio of sintered body containing colorant $\leq$ 0.68
B: 0.68 < The contrast ratio of sintered body containing colorant $\leq$ 0.71
C: The contrast ratio of sintered body containing colorant > 0.71

In the case of A, it has a high translucency which is sufficiently applicable to cases where high translucency is required. Among these, it is preferable to have a lower contrast ratio.

In the case of B, it has a translucency which is applicable to cases to some extent where high translucency is required.

In the case of C, translucency is low and it may be unsuitable for cases in which high translucency is required.

Example 1: Zirconia powder containing 5.5 mol% of solid-solved yttria (Zpex SMILE: manufactured by Tosoh Corporation, containing 0.05 mass% of alumina) was filled in a mold ($\varphi$100 mm), and press molding (surface pressure: 50 MPa) was performed to obtain a molded body. Further, the molded body was subjected to CIP treatment (maximum load pressure: 200 MPa, load pressure after releasing: 0 MPa, holding time: 1 minute, repeat times: 10 times). Thereafter, calcination was performed in an electric furnace (1000 °C, 30 minutes) to prepare a zirconia temporarily sintered body. The temporarily sintered body was immersed in an indium nitrate solution (indium concentration: 3.86 mass% as indium) at room temperature under an atmospheric pressure for 12 hours. Thereafter, the zirconia temporarily sintered body was taken out from the solution and completely dried in a dryer at 120 °C to prepare a zirconia mill blank for dental cutting and machining. A test specimen was prepared by cutting and machining the prepared zirconia mill blank for dental cutting and machining and was fired in a firing furnace (firing temperature: 1550 °C, temperature increase rate: 5 °C/min, holding time: 120 minutes) to prepare a zirconia sintered body.

Example 2: A zirconia sintered body was prepared in the same manner as in Example 1 except that an indium concentration of indium nitrate solution was 4.5 mass% (as indium).

Example 3: A zirconia sintered body was prepared in the same manner as in Example 1 except that an indium concentration of indium nitrate solution was 6.0 mass% (as indium).

Example 4: A zirconia sintered body was prepared in the same manner as in Example 1 except that an indium concentration of indium nitrate solution was 8.0 mass% (as indium).

Example 5: A zirconia sintered body was prepared in the same manner as in Example 1 except that an indium concentration of indium nitrate solution was 10.0 mass% (as indium).

Example 6: A zirconia sintered body was prepared in the same manner as in Example 1 except that an indium concentration of indium nitrate solution was 12.0 mass% (as indium).

Example 7: A zirconia sintered body was prepared in the same manner as in Example 1 except that a temporarily sintered body was immersed in an indium nitrate-yttrium nitrate solution (indium concentration: 4.10 mass% as indium, yttrium concentration: 3.07 mass% as yttrium) at room temperature under an atmospheric pressure for 12 hours. Example 8: A zirconia sintered body was prepared in the same manner as in Example 1 except that a mixed powder of a zirconia powder containing 5.5 mol% of solid-solved yttria (Zpex SMILE: manufactured by Tosoh Corporation, containing 0.05 mass% of alumina) and a zirconia powder containing 5.5 mol% of solid-solved yttria and iron oxide (Zpex Yellow: manufactured by Tosoh Corporation, containing 0.05 mass% of alumina) (mixing ratio (mass) 75:25) was used.

Example 9: Zirconia mixture powder prepared by mixing 93.2 g of zirconia powder containing 3.0 mol% of solid-solved yttria and 6.8 g of indium oxide in a ball mill, was filled in a mold ($\varphi$100 mm), and press molding (surface pressure: 50 MPa) was performed to obtain a molded body. Further, the molded body was subjected to CIP treatment (maximum load pressure: 200 MPa, load pressure after releasing: 0 MPa, holding time: 1 minute, repeat times: 10 times). Thereafter, calcination was performed in an electric furnace (1000 °C, 30 minutes) to prepare a zirconia temporarily sintered body. A test specimen was prepared by cutting and machining the prepared zirconia mill blank for dental cutting and machining and was fired in a firing furnace (firing temperature: 1550 °C, temperature increase rate: 5 °C/min, holding time: 120 minutes) to prepare a zirconia sintered body.

Example 10: A zirconia sintered body was prepared in the same manner as in Example 9 except that zirconia mixture powder prepared by mixing 95.5g of zirconia powder containing 4.0 mol% of solid solved yttria and 4.5g of indium oxide in a ball mill was used.

Example 11: A zirconia sintered body was prepared in the same manner as in Example 7 except that a concentration of indium nitrate-yttrium nitrate solution was 1.71 mass% of indium concentration as indium and 3.07 mass% of yttrium concentration as yttrium.

Comparative Example 1: Zirconia powder containing 5.5 mol% of solid-solved yttria (Zpex SMILE: manufactured by Tosoh Corporation, containing 0.05 mass% of alumina) was filled in a mold ($\phi$100 mm), and press molding (surface pressure: 50 MPa) was performed to obtain a molded body. Further, the molded body was subjected to CIP treatment (maximum load pressure: 200 MPa, load pressure after releasing: 0 MPa, holding time: 1 minute, repeat times: 10 times). Thereafter, calcination was performed in an electric furnace (1000 °C, 30 minutes) to prepare a zirconia temporarily sintered body. A test specimen was prepared by cutting and machining the prepared zirconia mill blank for dental cutting and machining and was fired in a firing furnace (firing temperature: 1550 °C, temperature increase rate: 5 °C/min, holding time: 120 minutes) to prepare a zirconia sintered body.

Comparative Example 2: Zirconia powder containing 5.5 mol% of solid-solved yttria (Zpex SMILE: manufactured by Tosoh Corporation, containing 0.05 mass% of alumina) was filled in a mold ($\varphi$100 mm), and press molding (surface pressure: 50 MPa) was performed to obtain a molded body. Further, the molded body was subjected to CIP treatment (maximum load pressure: 200 MPa, load pressure after releasing: 0 MPa, holding time: 1 minute, repeat times: 10 times). Thereafter, calcination was performed in an electric furnace (1000 °C, 30 minutes) to prepare a zirconia temporarily sintered body. The temporarily sintered body was immersed in an yttrium nitrate solution (yttrium concentration: 3.0 mass% as yttrium) at room temperature under an atmospheric pressure for 12 hours. Thereafter, the zirconia temporarily sintered body was taken out from the solution and completely dried in a dryer at 120 °C to prepare a zirconia mill blank for dental cutting and machining. A test specimen was prepared by cutting and machining the prepared zirconia mill blank for dental cutting and machining and was fired in a firing furnace (firing temperature: 1550 °C, temperature increase rate: 5 °C/min, holding time: 120 minutes) to prepare a zirconia sintered body.

Comparative Example 3: A zirconia sintered body was prepared in the same manner as in Comparative Example 2 except that an yttrium concentration of yttrium nitrate solution was 8.0 mass% (as yttrium).

Comparative Example 4: A zirconia sintered body was prepared in the same manner as in Comparative Example 2 except that an yttrium concentration of yttrium nitrate solution was 10.0 mass% (as yttrium).

Comparative Example 5: A zirconia sintered body was prepared in the same manner as in Comparative Example 1 except that a mixed powder of a zirconia powder containing 5.5 mol% of solid-solved yttria (Zpex SMILE: manufactured by Tosoh Corporation, containing 0.05 mass% of alumina) and a zirconia powder containing 5.5 mol% of solid-solved yttria and iron oxide (Zpex SMILE Yellow: manufactured by Tosoh Corporation, containing 0.05 mass% of alumina) (mixing ratio (mass) 75:25) was used.

Comparative Example 6: A zirconia sintered body was prepared in the same manner as in Example 2 except that a mixed powder of a zirconia powder containing 5.5 mol% of solid-solved yttria (Zpex SMILE: manufactured by Tosoh Corporation, containing 0.05 mass% of alumina) and a zirconia powder containing 5.5 mol% of solid-solved yttria and iron oxide (Zpex SMILE Yellow: manufactured by Tosoh Corporation, containing 0.05 mass% of alumina) (mixing ratio (mass) 75:25) was used.

Comparative Example 7: A zirconia sintered body was prepared in the same manner as in Comparative Example 6 except that an yttrium concentration of yttrium nitrate solution was 8.0 mass% (as yttrium).

Comparative Example 8: A zirconia sintered body was prepared in the same manner as in Comparative Example 6 except that an yttrium concentration of yttrium nitrate solution was 10.0 mass% (as yttrium).

Comparative Example 9: A zirconia sintered body was prepared in the same manner as in Example 1 except that an indium concentration of indium nitrate solution was 0.9 mass% (as indium).

Comparative Example 10: A zirconia sintered body was prepared in the same manner as in Example 9 except that 92.8 g of zirconia powder containing 3.0 mol% of solid solved yttria and 7.2 g of indium oxide were used.

Comparative Example 11: A zirconia sintered body was prepared in the same manner as in Example 7 except that a concentration of indium nitrate-yttrium nitrate solution was 1.71 wt% of indium concentration as indium and 4.30 wt% of yttrium concentration as yttrium.

Comparative Example 12: A zirconia sintered body was prepared in the same manner as in Example 9 except that 95.5 g of zirconia powder containing 5.5 mol% of solid solved yttria and 4.5 g of indium oxide were used.

Comparative Example 13: A zirconia sintered body was prepared in the same manner as in Example 6 except that zirconia powder containing 4.0 mol% of solid solved yttria was used.

Table 1 shows the indium oxide and yttrium oxide contents and the characteristic test results of the prepared zirconia mill blank for dental cutting and machining in Examples and Comparative Examples.

[Table 1]

| | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| Yttrium content (mol%) | 5.5 | 5.5 | 5.5 | 5.5 | 5.5 | 5.5 | 6.0 | 5.5 | 3.0 | 4.0 | 6.0 |
| Indium content (mol%) | 0.39 | 0.45 | 0.60 | 0.79 | 1.13 | 1.33 | 0.40 | 1.13 | 2.99 | 2.02 | 0.20 |
| Yttrium content (mol%) + Indium content (mol%) | 5.89 | 5.95 | 6.10 | 6.29 | 6.63 | 6.83 | 6.40 | 6.63 | 5.99 | 6.02 | 6.20 |
| Colorant | - | - | - | - | - | - | - | Fe | - | - | - |
| Contrast ratio | 0.66 | 0.64 | 0.63 | 0.62 | 0.62 | 0.65 | 0.64 | 0.65 | 0.67 | 0.64 | 0.65 |
| Evaluation | B | A | A | A | A | A | A | A | B | A | A |

| | Comparative Example | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example | Comparative Example 12 | Comparative Example 13 |
|---|---|---|---|---|---|---|---|---|---|---|---|---|---|
| Yttrium content (mol%) | 5.5 | 5.9 | 6.3 | 6.5 | 5.5 | 5.9 | 6.3 | 6.5 | 5.5 | 3.0 | 6.2 | 5.5 | 4 |
| Indium content (mol%) | 0.00 | 0.00 | 0.00 | 0.00 | 0.00 | 0.00 | 0.00 | 0.00 | 0.10 | 3.20 | 0.38 | 2.00 | 1.35 |

| | Compara-tive Exam-ple | Compara-tive Exam-ple 2 | Compara-tive Exam-ple 3 | Compara-tive Exam-ple 4 | Compara-tive Exam-ple 5 | Compara-tive Exam-ple 6 | Compara-tive Exam-ple 7 | Compara-tive Exam-ple 8 | Compara-tive Exam-ple 9 | Compara-tive Exam-ple 10 | Compara-tive Exam-ple | Compara-tive Exam-ple 12 | Compara-tive Exam-ple 13 |
|---|---|---|---|---|---|---|---|---|---|---|---|---|---|
| Yttrium content (mol%) + Indium content (mol%) | 5.50 | 5.90 | 6.30 | 6.50 | 5.50 | 5.90 | 6.30 | 6.50 | 5.60 | 6.20 | 6.58 | 7.50 | 5.35 |
| Colorant | - | - | - | - | Fe | Fe | Fe | Fe | - | - | - | - | - |
| Contrast ratio | 0.70 | 0.69 | 0.68 | 0.71 | 0.74 | 0.73 | 0.73 | 0.75 | 0.70 | 0.69 | 0.68 | 0.71 | 0.71 |
| Evaluation | C | C | C | C | C | C | C | C | C | C | C | C | C |

Examples 1 to 11 showed high translucency similar to an enamel layer of a natural tooth since the total amount of the yttrium compound and the indium compound contained was 5.5 mol% to 7.0 mol%.

Comparative Examples 1 to 13 did not have high translucency similar to an enamel layer of a natural tooth since the indium compound was not contained, or the total amount of the yttrium compound and the indium compound was less than 5.5 mol% or more than 7.0 mol%.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

Industrial applicability

The present invention relates to a zirconia mill blank for dental cutting and machining and a preparing method thereof, and is a technique which can be used in the dental field.

**Claims**

1.  A zirconia mill blank for dental cutting and machining, containing,

    an yttrium compound and an indium compound as a stabilizer, wherein,
    an amount of the yttrium compound is within a range of 3.0 mol% to 6.0 mol% in terms of oxide,
    an amount of the indium compound is within a range of 0.2 mol% to 3.0 mol% in terms of oxide, and
    a total amount of the yttrium compound and the indium compound is within a range of 5.5 mol% to 7.0 mol% in terms of oxide.

2.  The zirconia mill blank for dental cutting and machining according to claim 1, wherein,
    a sintered body of the zirconia mill blank for dental cutting and machining has a contrast ratio of 0.68 or less for a sample having a thickness of 1 mm.

3.  The zirconia mill blank for dental cutting and machining according to claim 1, wherein,

    the zirconia mill blank for dental cutting and machining further contains a colorant, and
    a sintered body of the zirconia mill blank for dental cutting and machining has a contrast ratio of 0.68 or less for a sample having a thickness of 1 mm.

4.  The zirconia mill blank for dental cutting and machining according to claim 1, wherein,

    the zirconia mill blank for dental cutting and machining contains no colorant, and
    a sintered body of the zirconia mill blank for dental cutting and machining has a contrast ratio of 0.65 or less for a sample having a thickness of 1 mm.

5.  A dental prosthetic device prepared by sintering the zirconia mill blank for dental cutting and machining according to any one of claims 1 to 4.

Europäisches
Patentamt

European
Patent Office

Office européen
des brevets

## EUROPEAN SEARCH REPORT

Application Number

EP 21 20 2787

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 611 150 A1 (SHOFU INC [JP]) 19 February 2020 (2020-02-19) * examples; tables * ----- | 1-5 | INV. C04B35/486 C04B35/488 C04B35/64 A61C13/00 C04B41/00 C04B41/50 C04B41/85 |
| X | EP 3 712 121 A1 (SHOFU INC [JP]) 23 September 2020 (2020-09-23) * examples; tables * ----- | 1-5 | |
| X | EP 3 636 621 A2 (SHOFU INC [JP]) 15 April 2020 (2020-04-15) * examples; tables * ----- | 1-5 | |
| A | US 2010/248936 A1 (YAMADA YOSHIHISA [JP] ET AL) 30 September 2010 (2010-09-30) * claims 7, 8 * ----- | 1-5 | |
| A | US 2007/197368 A1 (TSUKUMA KOJI [JP] ET AL) 23 August 2007 (2007-08-23) * para.[0026]; claim 2 * ----- | 1-5 | |
| X,P | EP 3 892 254 A1 (SHOFU INC [JP]) 13 October 2021 (2021-10-13) * whole doucment, but see in particular claim 4 * ----- | 1-5 | TECHNICAL FIELDS SEARCHED (IPC) C04B A61C |

The present search report has been drawn up for all claims

| Place of search | Date of completion of the search | Examiner |
|---|---|---|
| Munich | 22 April 2022 | Munro, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 2787

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2022

| Patent document cited in search report | | Publication date | Patent family member(s) | | Publication date |
|---|---|---|---|---|---|
| EP 3611150 | A1 | 19-02-2020 | CN | 110678435 A | 10-01-2020 |
| | | | EP | 3611150 A1 | 19-02-2020 |
| | | | JP | WO2018155459 A1 | 12-12-2019 |
| | | | KR | 20190122223 A | 29-10-2019 |
| | | | US | 2020113658 A1 | 16-04-2020 |
| | | | WO | 2018155459 A1 | 30-08-2018 |
| EP 3712121 | A1 | 23-09-2020 | CN | 111454056 A | 28-07-2020 |
| | | | EP | 3712121 A1 | 23-09-2020 |
| | | | JP | 2020117495 A | 06-08-2020 |
| | | | KR | 20200090640 A | 29-07-2020 |
| | | | US | 2020331807 A1 | 22-10-2020 |
| EP 3636621 | A2 | 15-04-2020 | CN | 110893123 A | 20-03-2020 |
| | | | EP | 3636621 A2 | 15-04-2020 |
| | | | JP | 2020033338 A | 05-03-2020 |
| | | | KR | 20200022348 A | 03-03-2020 |
| | | | US | 2020170753 A1 | 04-06-2020 |
| US 2010248936 | A1 | 30-09-2010 | JP | 2010220779 A | 07-10-2010 |
| | | | US | 2010248936 A1 | 30-09-2010 |
| US 2007197368 | A1 | 23-08-2007 | EP | 1820786 A1 | 22-08-2007 |
| | | | JP | 5125065 B2 | 23-01-2013 |
| | | | JP | 2007246384 A | 27-09-2007 |
| | | | US | 2007197368 A1 | 23-08-2007 |
| EP 3892254 | A1 | 13-10-2021 | CN | 113491591 A | 12-10-2021 |
| | | | EP | 3892254 A1 | 13-10-2021 |
| | | | JP | 2021165222 A | 14-10-2021 |
| | | | KR | 20210122703 A | 12-10-2021 |
| | | | US | 2021401553 A1 | 30-12-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010150063 A